# EUROPEAN PATENT APPLICATION

(11) **EP 0 550 964 A2**
(43) Date of publication of application: **14.07.1993**
(21) Application number: 92310727.0
(22) Date of filing: 24.11.1992
(51) Int. Cl.: C07C 2/18, C07C 2/26

(54) **Process for oligomerizing olefins using halogenated phosphorus-containing acid on montmorillonite clay**

(30) Priority: 16.12.1991 US 807342
(71) Applicant: TEXACO CHEMICAL COMPANY, Houston, Texas 77056 (US)
(72) Inventor: Sanderson, John Ronald, Leander, Texas 78641 (US); Knifton, John Frederick, Austin, Texas 78726 (US)
(74) Representative: Brock, Peter William

(57) **Abstract**

Olefins having 10 to 24 carbon atoms can be oligomerized to form products suitable as synthetic lubricants by contacting the olefins at elevated temperature with a catalyst comprising a halogenated phosphorus containing acid on a montmorillonite clay substrate.

## Description

The invention relates to the preparation of synthetic lubricant base stocks, and more particularly to synthetic lubricant base stocks made by oligomerizing linear olefins.

Synthetic lubricants are prepared from man-made base stocks having uniform molecular structures and, therefore, well-defined properties that can be tailored to specific applications. Mineral oil base stocks, on the other hand, are prepared from crude oil and are complex mixtures of naturally occurring hydrocarbons. The greater uniformity of synthetic lubricants generally results in superior properties, for example, excellent thermal stability. As automobile engines are reduced in size to save weight and fuel, they run at higher temperatures, and therefore require a more thermally stable oil. Because lubricants made from synthetic base stocks have such properties as excellent oxidative/thermal stability, very low volatility, and good viscosity indices over a wide range of temperatures, they offer better lubrication and permit longer drain intervals, with less oil vaporization loss between oil changes.

Synthetic base stocks may be prepared by oligomerizing internal and alpha-olefin monomers to form a mixture of dimers, trimers, tetramers, and pentamers, with minimal amounts of higher oligomers. The unsaturated oligomer products are then hydrogenated to improve their oxidative stability. The resulting synthetic base stocks have uniform isoparaffinic hydrocarbon structures similar to those of high quality paraffinic mineral base stocks, but have the superior properties mentioned above, because of their greater uniformity.

Synthetic base stocks are produced in a broad range of viscosity grades. It is common practice to classify base stocks by their viscosities, measured in centistokes (cSt) at 100°C. Base stocks with viscosities less than or equal to 4 cSt are "low viscosity" base stocks, whereas base stocks having a viscosity of 40 to 100 cSt are "high viscosity" base stocks. Base stocks having a viscosity of 4 to 8 cSt are "medium viscosity" base stocks. The low viscosity base stocks generally are recommended for low temperature applications. Higher temperature applications, such as motor oils, automatic transmission fluids, turbine lubricants, and other industrial lubricants, generally require higher viscosities, such as those provided by medium viscosity base stocks (i.e. 4 to 8 cSt grades). High viscosity base stocks are used in gear oils and as blending stocks.

The viscosity of the base stocks is determined by the length of the oligomer molecules formed during the oligomerization reaction. The degree of oligomerization is affected by the catalyst and reaction conditions employed during the oligomerization reaction. The length of the carbon chain of the monomer starting material also has a direct influence on the properties of the oligomer products. Fluids prepared from short-chain monomers tend to have low pour points and moderately low viscosity indices, whereas fluids prepared from long-chain monomers tend to have moderately low pour points and high viscosity indices. Oligomers prepared from long-chain monomers generally are more suitable than these prepared from shorter-chain monomers for use as medium viscosity synthetic lubricant base stocks.

One known approach to oligomerizing long-chain olefins to prepare synthetic lubricant base stocks is to contact the olefin with boron trifluoride, together with a promoter. See, for example, US-A-4400565, US-A-4420646, US-A-4420647 and US-A-4434308. However, boron trifluoride gas (BF₃) is a pulmonary irritant, and breathing the gas, or fumes formed by hydration of the gas with atmospheric moisture poses hazards which are preferably avoided. Additionally, the disposal/neutralization of BF₃ raises environmental concerns. Thus, methods for oligomerizing long-chain olefins using non-hazardous, non-polluting catalysts represent a substantial improvement in the art.

Recently we have filed a number of Applications relating to the oligomerization of olefins using non-polluting catalysts. These Applications include EP-A-0449453, EP-A-0459641, EP-A-0466305, EP-A-0466307, and US-A-0513963, which use various forms of catalyst derived from montmorillonite clay.

We have now found, surprisingly, that a high conversion of olefin into oligomer may be obtained by contacting the olefin with a catalyst prepared by depositing a halogenated phosphorus-containing acid on a substrate comprising montmorillonite clay. We have further discovered that when the halogenated phosphorus-containing acid is a fluorinated phosphorus-containing acid, the process of the present invention results in a very high percentage of trimer and higher oligomers, i.e., a very low dimer to trimer ratio. A high proportion of trimer and higher oligomers is particularly desirable when preparing a synthetic lubricant base stock from decene. In the absence of low dimer to trimer ratio obtained using the present invention, substantial amounts of decene dimer must be recycled and further oligomerized to prepare enough oligomers having sufficient molecular weight to obtain base stocks suitable for synthetic lubricants. In addition to being excellent catalysts, the treated clays of the present invention are less hazardous and more easily handled than BF₃.

The present invention provides a process for the preparation of oligomers by contacting linear olefins having 10 to 24 carbon atoms at elevated temperature with a catalyst comprising a halogenated phosphorus containing acid on a montmorillonite clay substrate.

Preferably, the halogenated, phosphorus-containing acid is a halogenated derivative of hypophosphorous acid, hypophosphoric acid, orthophosphoric acid, metaphosphoric acid, or polyphosphoric acid.

More preferably, the halogenated, phosphorus-containing acid is fluorophosphoric acid or difluorophosphoric acid.

The invention also provides a process for the preparation of oligomers by contacting linear olefins having 10 to 24 carbon atoms at elevated temperature with a heterogeneous catalyst comprising a silica gel alkylsulfonic acid, preferably one having the structure: 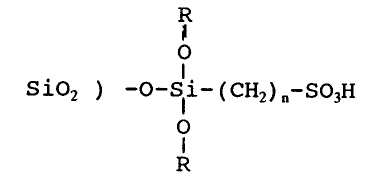
wherein R is an alkyl group having from 1 to 3 carbon atoms and n is 3 to 10.

We have discovered that an improved conversion of olefin into oligomer may be obtained where montmorillonite clays are treated with a halogenated, phosphorus-containing acid before use as an oligomerization catalyst.

The olefin monomer feedstocks used in the present invention may be compounds comprising (1) alpha-olefins having the formula R"CH=CH₂, where R" is an alkyl group having 8 to 22 carbon atoms, and (2) internal olefins having the formula RCH=CHR', where R and R' are the same or different alkyl groups having 1 to 21 carbon atoms, provided that the total number of carbon atoms in any one olefin is from 10 to 24, inclusive. Mixtures of internal and alpha-olefins may be used, as well as mixtures of olefins having different numbers of carbon atoms, provided that the total number of carbon atoms in any one olefin is from 10 to 24, inclusive. The alpha and internal-olefins to be oligomerized according to this invention may be obtained by processes well-known to those skilled in the art and are commercially available.

The oligomerization reaction may be represented by the following general equation: 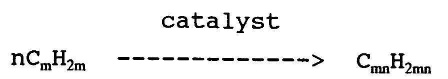
where n represents moles of monomer and m represents the number of carbon atoms in the monomer. Thus, the oligomerization of 1-decene may be represented as follows: 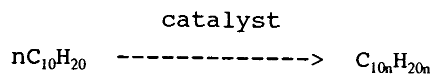
The reaction occurs sequentially. Initially, olefin monomer reacts with olefin monomer to form dimers. Most of the dimers that are formed then react with additional olefin monomer to form trimers, and so on. This results in an oligomer product distribution that varies with reaction time. As the reaction time increases, the olefin monomer conversion increases, and the selectivities for the heavier oligomers increase. Generally, each resulting oligomer contains one double bond.

The oligomers are prepared using montmorillonite clay which has been treated with a halogenated, phosphorus-containing acid.

Montmorillonite clays are one group of the class of smectite clays. Smectite clays have a small particle size and unusual intercalation properties that afford them a high surface area. Smectites comprise layered sheets of octahedral sites between sheets of tetrahedral sites. The distance between the layers can be adjusted by swelling, using an appropriate solvent. Three-layered sheet-type smectites include montmorillonites. The montmorillonite structure may be represented by the following formula: 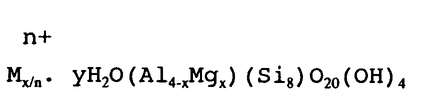
where M represents the interlamellar (balancing) cations, normally sodium or lithium; and x, y and n are integers.

Montmorillonite clays may be acid-activated by such mineral acids as sulphuric acid, and hydrochloric acid. Mineral acids activate montmorillonites by attacking and solubilizing structural cations in the octahedral layers. This opens up the clay structure and increases surface area. These acid-treated clays act as strong Bronsted acids. Illustrative examples of commercially available acid-treated clays include Engelhard Corporation's Grade F24, having a moisture content of 12 wt.%, a residual acidity of 16 mg KOH/g, and a surface area of 350 m²/g; Grade F124, having a moisture content of 4 wt.%, a residual acidity of 14 mg KOH/g, and a surface area of 350 m²/g; Grade F13, having a moisture content of 12 wt.%, a residual acidity of 15 mg KOH/g, and a surface area of 300 m²/g; Grade F113, having a moisture content of 4 wt.%, a residual acidity of 15 mg KOH/g, and a surface area of 300 m²/g; and Grade F224, having virtually no moisture, and having a residual acidity of 5 mg KOH/g, and a surface area of 350 m²/g.

We have discovered that a high conversion of olefin into oligomer may be obtained by contacting the olefin feed with a catalyst prepared by depositing a halogenated, phosphorus-containing acid on a substrate comprising a montmorillonite clay. The montmorillonite substrate may comprise a neutral to basic clay (i.e. having a pH of 7 or more), or one that has previously been acid treated as described above. Preferably, the clay has not been treated with an acid before use as a substrate for the halogenated, phosphorus-containing acid, and has a residual acidity of less than 1 mg KOH/g. An especially preferred clay is Engelhard's Grade F2C, having a moisture content at 5°C of 15 wt.% and a pH of 7.5. Another suitable commercially available clay is Engelhard's Desiccate-25.

In the present invention, the clay is treated with halogenated, phosphorus-containing acid before the oligomerization reaction. It is preferred that the halogenated, phosphorus-containing acid deposited on the montmorillonite clay is a fluorinated, phosphorus-containing acid, for example, the fluorinated derivatives of the following acids: hypophosphorous acid, hypophosphoric acid, orthophosphoric acid, metaphosphoric acid, and polyphosphoric acid. It is especially preferred that the halogenated, phosphorus-containing acid to be deposited on the montmorillonite clay is difluorophosphoric acid or fluorophosphoric acid.

The clay is conveniently added to a solution of 2 to 100 wt.%, preferably from 5 to 20 wt.%, of the halogenated, phosphorus-containing acid in water or an organic solvent, such as acetone or alcohol. The ratio of clay to halogenated, phosphorus-containing acid solution should be sufficient to provide a catalyst having a quantity of phosphorus deposited thereon ranging from 0.1 to 20 wt.%, preferably 1 to 5 wt.%. The clay should remain in the halogenated, phosphorus-containing acid solution for a period of time, and under agitation to the extent necessary to meet these requirements, and then filtered and dried. Optionally, the filtered clay having said acid deposited thereon may be washed with distilled water and then dried, preferably under mild conditions.

Preferably, the halogenated, phosphorus-containing acid treated catalyst is heat treated before running the reaction. We found that heat treatment of the catalyst before running the oligomerization reaction makes the ctalayst more active and produces a higher olefin conversion. Additionally, clays heat treated in this manner are more stable, remaining active during the oligomerization reaction over a longer period. The clays may be heat treated at temperatures of 50 to 400°C, with or without the use of a vacuum. A more preferred temperature range is 50 to 300°C. Optionally, an inert gas may be also used during heat treatment. Preferably, the clay should be heat treated under conditions and for a length of time which will reduce the water content of the clay to approximately 1 to 2 wt.%, or less.

The oligomerization reaction may be carried out either batchwise, in a stirred slurry reactor, or continuously, in a fixed bed continuous flow reactor. The catalyst concentration should be sufficient to provide the desired catalytic effect. The temperatures at which the oligomerization may be performed are generally between 50 to 300°C, with the preferred range being 120 to 250°C, and the especially preferred range being 160 to 180°C, for optimum conversion. However, to obtain a lower dimer to trimer ratio, it is preferred that the oligomerization is performed at a temperature of 135 to 160°C, and especially preferred that the temperature is from 135 to 145°C.

At reaction temperatures of 200°C or higher, the amount of unsaturation remaining in the products of the oligomerization reaction may decrease, thus reducing the degree of hydrogenation necessary to remove unsaturation from the base stocks (see below). However, at temperatures above 200°C, the olefin conversion may decrease and the dimer to trimer ratio will increase. We have found that the addition of a hydrocarbon containing a tertiary hydrogen, such as methylcyclohexane, may further reduce the amount of unsaturation present in the base stocks. One skilled in the art may choose the reaction conditions most suited to the results desired for a particular application. The reaction may be run at pressures of from 0.1 to 7.0 MPa (0 to 1000 psig).

Furthermore, we have discovered surprisingly, that a substantially higher dimer/trimer ratio may be obtained by contacting the olefin feed with a catalyst comprising a silica gel alkylsulphonic acid. We believe it was heretofore unknown in the art to use silica gel alkylsulphonic acids to prepare synthetic lubricant base stocks having a very high percentage of dimers. By maintaining a low percentage of trimer and higher oligomers in the reaction product, Applicants are able to obtain base stocks having excellent low temperature properties while using long-chain monomers as feedstock.

The invention further relates, therefore, to a process for the preparation of synthetic lubricant base stocks having a high dimer to trimer ratio, comprising contacting linear olefins containing from 14 to 24 carbon atoms with a silica gel alkylsulphonic acid catalyst preferably having the formula: 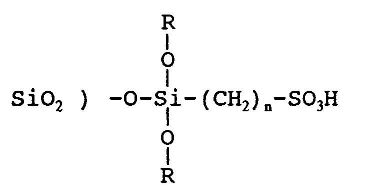
wherein R is alkyl having 1 to 3 carbon atoms and n is 3 to 10. This will provide a product having a dimer to trimer ratio of 5:1 or higher. It is also possible to use catalysts with alkylsulphonic acid groups bonded to other oxides from Group III or IV of the Periodic Table.

The catalysts used to effect this reaction are silica gel alkylsulphonic acids. As used in this application, the term "silica gel alkylsulphonic acids" means silica having alkylsulphonic acid groups chemically bound thereto. In other words, the alkylsulphonic acids are not merely deposited on the silica, but covalently bonded to the silica. Other catalysts within the scope of the present invention process include alkylsulphonic acids bound to the other Group IV oxides, such as titania, zirconia, and the like, or bound to Group III oxides, such as alumina, and the like.

Preferably, the silica gel alkylsulphonic acids used in this embodiment of the present invention have the formula: 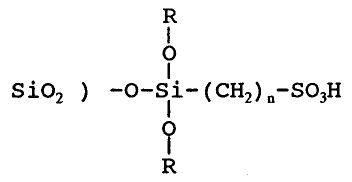
wherein R is alkyl having 1 to 3 carbon atoms and n is 3 to 10. More preferably, the silica gel alkylsulphonic acid used in the present invention is silica gel propylsulphonic acid. The preparation of silica-bound sulphonic acids is exemplified herein by the preparation of silica gel propylsulphonic acid.

Silica gels are commercially available in at least the following Tyler Standard mesh sizes: 3-8 (2.38-6.73 mm); 6-16 (1.19-3.36 mm); 14-20 (0.841-1.41 mm); 14-42 (0.354-1.41 mm); and 28-200 (0.074-0.595 mm) and greater. A suitable commercially available silica gel is the grade 12, 28-200 mesh (0.074-0.595 mm) silica gel available from Aldrich Chemical Co., Inc. Silica gel propylsulphonic acid may be prepared by treating silica gel with (3-mercaptopropyl )trimethoxysilane. The resulting surface-modified mercaptan is then oxidized using aqueous H₂O₂, to give the silica-bound sulphonic acid. 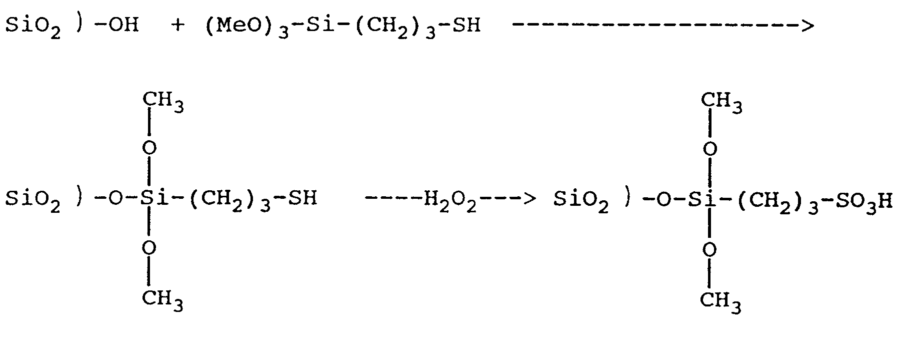 This and other procedures are more fully described by R.D. Badley and W.T. Ford, in "Silica-Bound Sulfonic Acid Catalysts", J. Org. Chem., vol. 54, no. 23, pages 5437-5443 (1989), and in the Examples of this Application.

The dimerization reaction may be carried out in either a stirred slurry reactor or in a fixed bed continuous flow reactor. The catalyst concentration should be sufficient to provide the desired catalytic effect. The temperatures at which the dimerization may be performed are between 50 and 300°C, with the preferred range being from 140 to 180°C. It is especially preferred that the temperature is from 140 to 160°C.

At reaction temperatures of 200°C or higher, the amount of unsaturation remaining in the products of the oligomerization reaction may decrease, thus reducing the degree of hydrogenation necessary to remove unsaturation from the base stocks. However, temperatures above 200°C may adversely affect olefin conversion and the dimer to trimer ratio. We have found that the addition of a hydrocarbon containing a tertiary hydrogen, such as methylcyclohexane, may further reduce the amount of unsaturation present in the base stocks. One skilled in the art may choose the reaction conditions most suited to the results desired for a particular application. The reaction may be run at pressures of 0.1 to 7.0 MPa (0 to 1000 psig).

Following the oligomerization reaction, the unsaturated oligomers may be hydrogenated to improve their thermal stability and to guard against oxidative degradation during their use as lubricants. The hydrogenation reaction for 1-decene oligomers may be represented as follows: 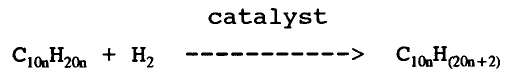
wherein n represents moles of monomer used to form the oligomer. Hydrogenation processes known to those skilled in the art may be used to hydrogenate the oligomers. A number of metal catalysts are suitable for promoting the hydrogenation reaction, including nickel, platinum, palladium, copper, and Raney nickel. These metals may be supported on a variety of porous materials, such as kieselguhr, alumina, or charcoal, or they may be formulated into a bulk metal catalyst. A particularly preferred catalyst for this hydrogenation is a nickel-copper-chromia catalyst described in US-A-3152998. Other U.S. Patents disclosing known hydrogenation procedures include US-A-4045508, US-A-4013736, US-A-3997621 and US-A-3997622.

Unreacted monomer may be removed either before or after the hydrogenation step. Optionally, unreacted monomer may be stripped from the oligomers before hydrogenation and recycled to the catalyst bed for oligomerization. The removal or recycle of unreacted monomer or, if after hydrogenation, the removal of non-oligomerized alkane, should be conducted under mild conditions using vacuum distillation procedures known to those skilled in the art. Similarly, when the olefin feed is decene, it also may be necessary to remove or recycle decene dimers in order to obtain base stocks suitable for synthetic lubricants. The extent of removal or recycle of decene dimers is minimized by the preferred embodiments of the present invention. For example, we have demonstrated that when decene is oligomerized in the presence of difluorophosphoric acid or fluorophosphoric acid catalysts at a temperature up to 180°C, dimer to trimer+ ratios less than 0.80 are routinely observed. At slightly lower temperatures, we obtained ratios of dimers to trimers and higher oligomers of less than 0.70, and as low as less than 0.50.

Distillation at temperatures exceeding 250°C may cause the oligomers to break down in some fashion and come off as volatiles. Preferably, therefore, the reboiler or pot temperature should be kept at or below 225°C when stripping out the monomer. Procedures known by those skilled in the art to be alternatives to vacuum distillation also may be employed to separate unreacted components from the oligomer.

The invention will be further illustrated by the following Examples.

### EXAMPLES 1 TO 15

In the Examples 1 to 15 detailed in Table 1 below, the following procedures were used:

### Preparation of Catalysts

A. Difluorophosphoric acid on Grade F2C
   To 100 g of montmorillonite clay (Engelhard Clay Grade F2C powder) was added, under nitrogen, a solution of difluorophosphoric acid (10.0g) in distilled water (100 cm³). The mixture was stirred under nitrogen for 24 hours. The white solid product was recovered by filtration, washed with distilled water until the washings were pH neutral, and dried in vacuo at 40°C for 4 hours, and then at 150°C overnight.
   Analysis of the product showed:
   % water = 1.25
   Acidity = 0.07 meg/g
B. Difluorophosphoric acid on Desiccate-25
   To 200 g of clay granules (Engelhard Desiccate-25) was added, under nitrogen, a solution of difluorophosphoric acid (20 g) in distilled in water (200 g). The mixture was stirred under nitrogen for 24 hours. The white solids were recovered by filtration, washed with distilled water until the washings were pH neutral, and dried in vacuo at 40°C for 4 hours, and then at 150°C overnight.
   Analysis of the product showed:
   % water = 1.26
   Acidity = 0.053 meg/g

### Olefin Oligomerization

Olefin and catalyst were charged to a flask equipped with a stirrer, thermometer, heating mantle, condenser, and nitrogen purge. The mixture was heated at the desired temperature, for the desired time, with vigourous stirring. At the end of the reaction, the mixture was cooled to ambient temperature, filtered with suction and the liquid effluent was analyzed by liquid chromatography. The results are shown in Table 1 below.

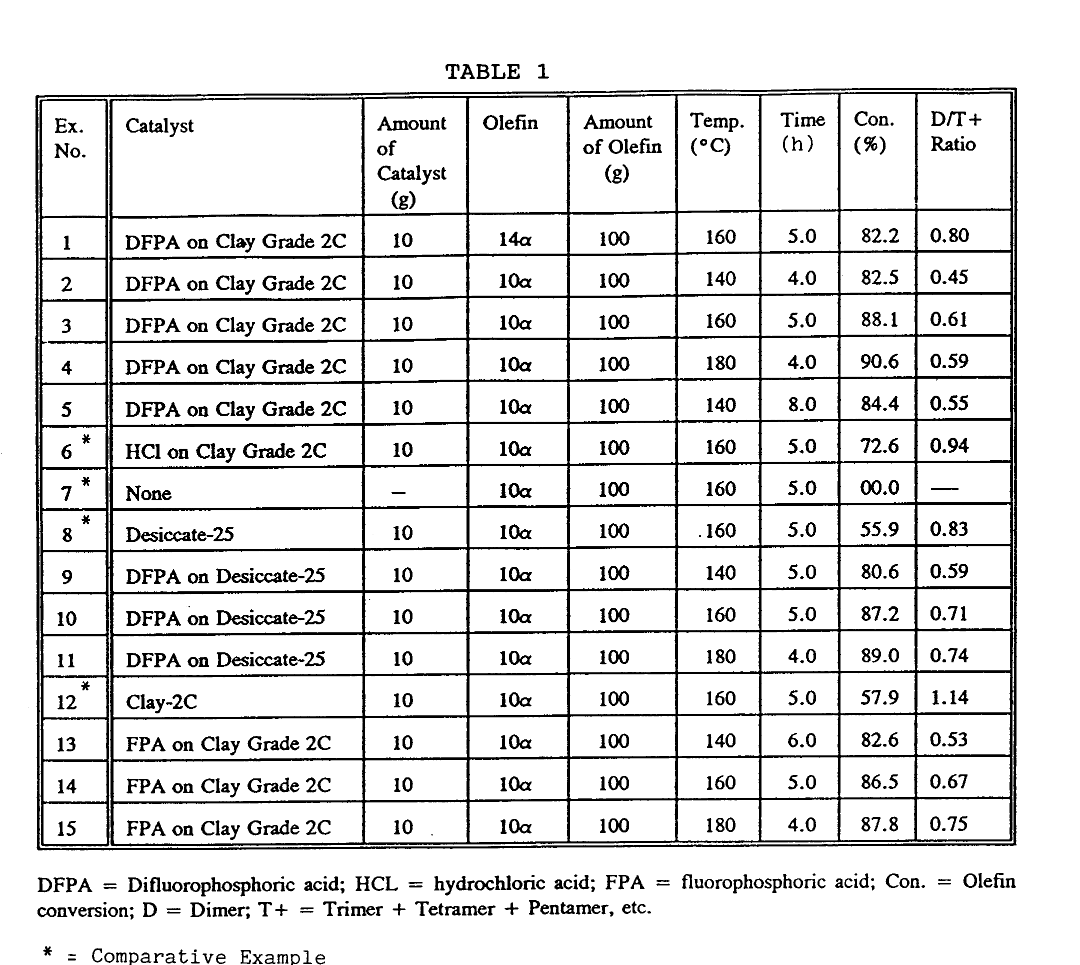

### EXAMPLES 16 TO 27

In the Examples 16 to 27, the following procedure was used:

### Catalyst Preparation

Silica gel (500 g) and 10 % HCl (1000 g) were refluxed for 4.0 hours. The solids were collected with suction and washed with water until the washings were neutral to litmus. The solid was then dried at 100°C in a vacuum oven overnight.

500 g of the above silica gel was treated with 1000 g of toluene and refluxed for 5.0 hours. (A Dean-Stark trap was used to remove the small amount of water remaining). The trap was removed and 125 g of (3-mercaptopropyl) trimethoxysilane was added. The mixture was refluxed for 25 to 30 hours, and then cooled to ambient temperature. The solid was collected with suction and washed with toluene followed by acetone. The solids were dried in a vacuum oven at 100°C overnight.

To 500 g of the mercaptopropyl silica gel was slowly added 400 g water and 1500 g of 30% hydrogen peroxide. The slurry was stirred slowly overnight, and then let stand over the weekend. The solids were then collected with suction and washed with water and acetone, toluene, and then acetone once more. Finally the solids were dried in a vacuum oven overnight at 100°C. The dried material has the following analysis.
Acidity: 20.2 mg/g
Sulphur: 1.7 %
Water: 0.82 %

### Olefin Oligomerization

Olefin and catalysts were charged to a flask equipped with a stirrer, thermometer, heating mantle, condenser, and nitrogen purge. The mixture was heated to the desired temperature, for the desired time, with vigorous stirring. At the end of the reaction, the mixture was cooled to ambient temperature, filtered with suction, and the liquid effluent analyzed by liquid chromatography. The results are shown in Table 2 below.

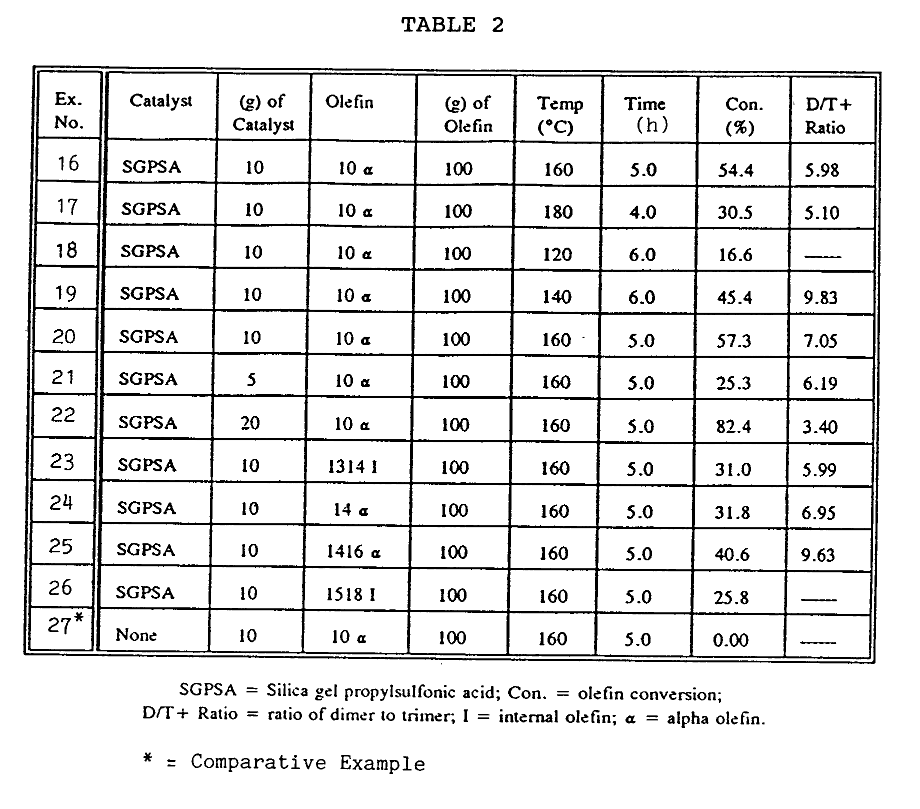

## Claims

1. A process for the preparation of oligomers by contacting linear olefins having 10 to 24 carbon atoms with a catalyst at elevated temperature characterised in that the catalyst comprises a halogenated phosphorus containing acid on a montmorillonite clay substrate.

2. A process according to Claim 1, characterised in that the elevated temperature is from 50 to 300°C.

3. A process according to Claim 2, characterised in that the temperature is from 135 to 160°C.

4. A process according to Claim 2, characterised in that the temperature is from 160 to 180°C.

5. A process according to any one of Claims 1 to 4, characterised in that the montmorillonite clay has a residual acidity of less than 1 mg KOH/g before treatment with the halogenated, phosphorus-containing acid.

6. A process according to any one of Claims 1 to 5, characterised in that the montmorillonite clay has a pH of 7 or more before treatment with the halogenated, phosphorus-containing acid.

7. A process according to any one of Claims 1 to 6, characterised in that the halogenated, phosphorus-containing acid is a halogenated derivative of hypophosphorous acid, hypophosphoric acid, orthophosphoric acid, metaphosphoric acid, or polyphosphoric acid.

8. A process according to Claim 7, characterised in that the halogenated, phosphorus-containing acid is fluorophosphoric acid or difluorophosphoric acid.

9. A process for the preparation of oligomers by contacting linear olefins having 10 to 24 carbon atoms with a catalyst at elevated temperature characterised in that the catalyst is a heterogeneous catalyst comprising a silica gel alkylsulfonic acid.

10. A process according to Claim 9, characterised in that the silica gel alkylsulfonic acid has the structure: 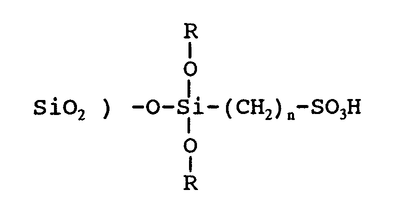 wherein R is an alkyl group having from 1 to 3 carbon atoms and n is 3 to 10.
